# FASCICULE DE BREVET EUROPEEN

(11) **EP 3 908 267 B1**
(45) Date de publication et mention de la délivrance du brevet: **26.02.2025**
(21) Numéro de dépôt: 20700705.5
(22) Date de dépôt: 13.01.2020
(51) Int. Cl.: A61K 31/047, A61K 31/78, A61K 35/74, A61K 35/741, A61K 35/744, A61K 9/00, A61P 17/04, A61P 17/06, A61P 17/08, A61P 17/10

(54) **PRODUIT POUR LE TRAITEMENT DE DYSBIOSES**
PRODUKT ZUR BEHANDLUNG VON DYSBIOSEN
PRODUCT FOR THE TREATMENT OF DYSBIOSES

(30) Priorité: 11.01.2019 FR 1900279
(43) Date de publication de la demande: 17.11.2021
(73) Titulaire: Cosmosoft, 75017 Paris (FR)
(72) Inventeur: GREFF, Daniel, 78490 Mere (FR)
(74) Mandataire: Cabinet Beau de Loménie
(86) Numéro de dépôt international: PCT/EP2020/050717
(87) Numéro de publication internationale: WO 2020/144380

(56) Documents cités:
- WO-A1-97/30692
- WO-A1-98/03152
- FR-A1- 2 703 907
- FR-A1- 2 879 452
- FR-A1- 2 913 337
- FR-A1- 2 918 884
- FR-A1- 2 918 886
- FR-A1- 2 973 701
- ANONYMOUS: "Osmo Soft Brûlures & Coups de soleil tube de 50 g ou de 150 g", 1 October 2015 (2015-10-01), XP055641792, Retrieved from the Internet <URL:https://www.cooper.fr/sites/default/files/NOTICE%20OSMOSOFT.pdf> [retrieved on 20191112]

## Description

### Domaine Technique

La présente invention concerne un produit cosmétique ou pharmaceutique pour le traitement topique de désordres ou de pathologies liés à un déséquilibre du microbiote cutané.

### Technique antérieure

La surface de la peau est colonisée en permanence par une très grande variété de micro-organismes constituant le microbiote cutané. Ce microbiote comprend notamment des bactéries, des levures et des parasites. Des dysbioses se traduisant par un déséquilibre entre les différentes colonies de microorganismes peuvent résulter d'une augmentation ou une réduction de la diversité bactérienne, par l'émergence de bactéries pathogènes ou d'un dérèglement de la réponse immunitaire. Des bactéries dites commensales présentes dans la flore résidente ont donc pour fonction de protéger l'hôte contre les infections déclenchées par la colonisation de microorganismes saprophytes devenus pathogènes. Certaines formes d'eczéma par exemple sont déclenchées par ces infections.

L'eczéma évolue par poussées, chaque poussée comprenant quatre phases. La première phase dite érythémateuse est caractérisée par des zones rouges mal délimitées et des démangeaisons. C'est au cours de cette phase que le prurit est le plus intense. A cette première phase succède la phase vésiculeuse. Les vésicules apparaissent sur les lésions érythémateuses : elles sont transparentes, renferment une sérosité claire et peuvent confluer pour former des bulles. Au cours d'une troisième phase dite phase de suintement, les vésicules se rompent, laissant s'écouler un liquide séreux jaunâtre, la peau se couvre alors de petites croûtes jaunâtres, résultat de la coagulation de la sérosité. Lorsque les croûtes sont tombées, le tégument prend un aspect érythémateux et lisse. La surface de la peau se craquelle et se recouvre de squames fines. C'est la phase de desquamation. Dans certains cas, l'ouverture des vésicules provoquent une mise à nu du derme.

Bien que l'antibiothérapie générale soit parfois indiquée en cas de surinfection, la plupart des eczémas sont traités par voie topique avec prescription de bains antiseptiques, et d'une corticothérapie locale, accompagnée d'une crème hydratante pour traiter la sécheresse de la peau, qui est un symptôme récurrent de ces dermatoses. Les antibiotiques et les antiseptiques peuvent freiner la colonisation des pathogènes, mais ne permettent pas de reconstituer le microbiote. De plus, les antihistaminiques par voie orale peuvent être nécessaires dans certains cas pour calmer les démangeaisons. Cependant, l'usage des dermocorticoïdes n'est pas sans effet secondaire: ils peuvent atrophier l'épiderme et le derme, et déclencher de l'acné ou une dermite périorale. Il est donc souhaitable de proposer un traitement de la dermatite atopique alternatif sans prescription de dermocorticoïdes.

Dans les traitements classiques prescrits dès la phase érythémateuse de la dermatite atopique, l'usage d'antiseptiques en solution aqueuse ou alcoolique est suivi de l'application de produits à base dermocorticoïdes. De plus, les personnes présentant un terrain allergique sont très fréquemment intolérantes à des substances irritantes telles que les conservateurs par exemple. Les traitements classiques sont donc potentiellement irritants, allergisants, et nécessitent des applications quotidiennes. Le Dupixent est un traitement alternatif administré en deux fois par mois seulement, mais il doit être injecté et son coût est élevé.

Il est donc souhaitable de disposer d'un produit pour le traitement de l'eczéma par voie topique qui présente au moins l'un des avantages suivants : être moins coûteux, être dépourvu de substances irritantes tels que des conservateurs, être dépourvu de dermocorticoïdes, et être moins contraignant à appliquer.

L'utilisation de matériel biologique a été proposée comme alternative aux traitements médicamenteux de l'eczéma et d'autres dermatoses telles que la rosacée et les états pelliculaires du cuir chevelu. Des extraits de bactéries ou des molécules synthétisées par des bactéries ont été incorporés dans des crèmes ou des lotions pour stimuler le métabolisme de certaines bactéries du microbiote résident altéré, ou pour lutter contre des bactéries pathogènes exogènes ou résidentes ayant acquis un caractère pathogène. Quatre exemples de ce type de traitement peuvent être donnés. Le premier concerne des extraits de bactéries filamenteuses non photosynthétiques et non fructifiantes introduits dans des crèmes pour renforcer la diversité de la flore cutanée résidente (FR 2 918 886 et FR 2 918 884). Le deuxième porte sur des produits cosmétiques anti-âge comprenant des bactéries probiotiques Lactobacillus et Bifidobacterium qui stimulent la flore existante. L'ajout, dans ces soins dermatologiques, de molécules actives naturellement produites par un microbiote sain pour stimuler la production par les peaux atopiques de peptides antimicrobiens à destination des pathogènes, constitue un troisième exemple de traitement de dermatose mettant en oeuvre un matériel biologique. Enfin, l'incorporation de prébiotiques pour nourrir la flore bactérienne résidente en est un quatrième. Le besoin subsiste néanmoins d'améliorer l'efficacité de tels traitements.

La présente invention met en oeuvre un gel comprenant de l'eau, de la glycérine, au moins un polyacrylate réticulé, et éventuellement un polyol différent de la glycérine, de préférence un diol. Un de ces gels a déjà été proposé pour le traitement cicatrisant de différentes affections cutanées dans la demande WO 2012/136934. Dans le cas de l'eczéma, l'effet cicatrisant du gel sur des lésions dermiques qui se sont formées au stade vésiculeux de la maladie a été rapportée. Cette même composition est décrite dans la demande WO 97/30692 pour ses propriétés microbicides et son utilisation dans le traitement de l'acné, des pellicules, de l'impétigo, des mycoses et infections microbiennes en général, dans la demande FR 2 703 907 pour son utilisation dans l'hydratation des couches superficielles de l'épiderme ou des cheveux, et dans la demande WO 98/03152 pour ses propriétés antimicrobiennes, hydratantes, cicatrisantes et anti-inflammatoires. Par ailleurs, le produit commercial Osmo Soft Brûlures & Coups de soleil a été proposé pour le traitement des brûlures superficielles grâce à ses propriétés apaisantes, anti-rougeurs, hydratantes et cicatrisantes.

La demande FR 2 879 452 a décrit l'utilisation d'un extrait de bactérie filamenteuse Vitreoscilla filiformis pour normaliser la flore microbienne de la peau, du cuir chevelu et/ou des muqueuses, en particulier pour leur utilisation dans le traitement des dermatites atopiques, de l'eczéma, de l'acné et des états pelliculaires ou séborrhéiques. Enfin, la demande FR 2913337 a proposé un procédé cosmétique pour le traitement de maladies de la peau tel que l'eczéma ou la rosacée, qui consiste à mélanger de façon extemporanée un extrait bactérien lyophilisé à une base cosmétique, puis à appliquer le mélange sur la peau. Cette méthode est présentée comme une alternative aux compositions prêtes à l'emploi contenant le microorganisme vivant.

Le besoin subsiste cependant de disposer d'un traitement de l'eczéma efficace à un stade plus précoce de la maladie.

### Exposé de l'invention

L'invention concerne un ensemble pour le traitement d'une dysbiose par voie topique comprenant un produit cosmétique ou un produit pharmaceutique, ledit produit ayant un pH compris 5.5 et 6.5 et comprenant essentiellement de l'eau, du glycérol, au moins un polyacrylate réticulé et au moins un polyol différent du glycérol. Dans un mode de réalisation, le produit comprend au moins 90% en masse d'eau, de glycérol, d'au moins un polyacrylate réticulé, d'un polyéthylèneglycol et de 1,2-octanediol. L'ensemble permet avantageusement de restaurer le microbiote cutané d'une peau saine, par action conjointe d'une composition de transplantation de matériel biologique

Selon la nature de la dysbiose, comme notamment sa nature, son intensité ou son stade d'avancement, le produit peut avoir soit une action cosmétique, à savoir améliorer l'apparence de la peau et/ou la sensation de confort de la personne lorsque la dysbiose est de nature non pathologique, soit avoir une action pharmaceutique, à savoir traiter une peau malade lorsque la dysbiose est de nature pathologique. La dysbiose n'est pas nécessairement une maladie, et le produit est un produit pharmaceutique uniquement lorsqu'il a une action thérapeutique et a vocation à traiter des conditions pathologiques de la peau. Un produit cosmétique n'a pas d'activité pharmaceutique. Ledit traitement peut comprendre une première étape d'application du produit sur la peau, suivie d'une deuxième étape de nettoyage dudit produit après l'avoir laisser agir pendant une durée suffisante. Au cours d'une troisième étape, on peut appliquer sur la peau une composition comprenant du matériel biologique en quantité suffisante pour rééquilibrer le microbiote et assainir la peau. Le produit est utilisé par exemple pour le traitement de la dermatite atopique.

Il a été découvert de façon surprenante que l'ensemble selon l'invention permet d'optimiser le traitement d'une dermatose par apport de matériel biologique, par exemple par transplantation de microbiote, en vue de restaurer un microbiote cutané sain. L'ensemble de l'invention permet avantageusement de réduire voire de supprimer l'usage d'antiseptiques et de crèmes stéroïdes pourtant nécessaires dans l'art antérieur pour soigner les inflammations cutanées.

On entend par « microbiote cutané » la flore microbienne qui se trouve essentiellement à la surface de la peau mais que l'on peut aussi trouver dans les follicules pileux, dans les glandes sébacées, dans les glandes sudoripares et dans le derme. Cette flore cutanée résidente comprend un ensemble de microorganismes parmi lesquels des bactéries, des levures, des champignons, des levures et des virus. Des bactéries dites commensales protègent l'hôte contre les infections déclenchées par la colonisation de microorganismes pathogènes. Les fréquences relatives des différentes espèces de microorganismes varient d'un individu à l'autre. Chez un même individu, il évolue notamment avec l'âge, l'alimentation, le pH, le taux d'humidité et la composition lipidique de la peau, la zone anatomique de la peau, la température corporelle, les agressions mécaniques et chimiques que subit la peau, les conditions climatiques auxquelles la peau est exposée, et les traitements médicamenteux administrés à l'individu. Le microbiote de la peau saine au sens de l'invention est défini comme étant celui d'une zone cutanée donnée : il est stable dans le temps pour une personne donnée ne souffrant pas d'une dermatose. Le microbiome cutané englobe le microbiote, ses gènes et son mode de fonctionnement. Le microbiome de la peau saine protège notamment des pathogènes invasifs, mais également d'un dessèchement de la peau.

Les bactéries dites commensales colonisent la peau sans être pathogènes et la protègent. Elles ne peuvent vivre qu'au contact de l'hôte, se nourrissent de matières organiques mortes et participent à leur dégradation. Des bactéries dites saprophytes, vivent au dépend de l'hôte, ne donnent pas de maladies chez un sujet sain mais peuvent devenir pathogènes chez un sujet immunodéprimé. Une même bactérie peut être à la fois commensale, saprophyte et pathogène.

Les bactéries commensales protègent l'hôte contre une colonisation par des bactéries pathogènes ou devenues pathogènes en sécrétant des bactériocines. Elles entrent en effet en compétition avec les bactéries invasives pour la consommation des nutriments. Au sens de l'invention, les bactéries commensales d'un microbiote sain ou d'un microbiote altéré, perturbé ou déséquilibré regroupent notamment Staphylococcus epidermidis, Staphylococcus hominis (bactéries commensales prépondérantes), Streptococcus mitis, Propionibacterium acnes, des espèces de Corynebacterium, Acinetobacter johnsoni (bactéries commensales fréquentes), et Staphylococcus warneri (bactéries commensales peu fréquentes). D'autres bactéries font également partie de ces microbiotes, comme Staphylococcus haemolyticus, Staphylococcus aureus, Corynebacterium lipophiles, Corynebacterium urealyticum, Corynebacterium minutissimum, Corynebacterium jeikeium, Propionibacterium avidum, Propionibacterium granulosum, Propionibacterium propionicum, Micrococcus luteus, Micrococcus varians, Streptococcus des groupes A, B et G, et les bactéries du genre Brevibacterium.

On entend par « dysbiose » une anomalie dans la composition bactérienne du microbiote, ou encore un déséquilibre, une altération ou une perturbation du fonctionnement du microbiote cutané. La dysbiose peut résulter d'une augmentation ou d'une réduction de la diversité bactérienne, de l'augmentation ou de la réduction d'une colonie d'espèce bactérienne résidente, ou de l'émergence d'une colonie bactérienne pathogène par exemple. La dysbiose peut être une pathologie ou un désordre altérant l'esthétique et le confort de la personne atteinte, ce désordre n'ayant pas un caractère pathologique. Une augmentation ou une réduction de la diversité bactérienne peut favoriser l'émergence de bactéries pathogènes et le développement de pathologies inflammatoires cutanées.

### Description des modes de réalisation

L'invention a pour objet un ensemble d'un produit et d'une composition conditionnés de façon séparée, pour son utilisation dans le traitement par voie topique d'une zone de peau d'une personne atteinte d'une dysbiose:
- ledit produit étant dépourvu d'agent conservateur, ayant un pH compris 5.5 et 6.5 et comprenant au moins 90% en masse d'un mélange constitué d'eau, de glycérol, d'au moins un polyacrylate réticulé, et d'au moins un polyol différent du glycérol,
- ladite composition étant une composition de transplantation bactérienne comprenant au moins un matériel biologique choisi dans le groupe constitué par une bactérie, un extrait d'une bactérie ou un extrait d'un milieu de culture d'une bactérie.

La présente demande décrit un produit pharmaceutique pour son utilisation dans le traitement par voie topique d'une zone de peau d'une personne atteinte d'une dysbiose, ledit traitement comprenant :
- une première étape d'application du produit sur la zone de peau, ledit produit étant dépourvu d'agent conservateur, ayant un pH compris 5.5 et 6.5 et comprenant au moins 90% en masse d'un mélange constitué d'eau, de glycérol, d'au moins un polyacrylate réticulé, et d'au moins un polyol différent du glycérol,
- une deuxième étape de nettoyage de la zone de peau de manière à éliminer le produit qui a été appliqué à l'étape précédente,
- une troisième étape de transplantation, sur la zone de peau nettoyée, d'une composition de transplantation bactérienne comprenant au moins un matériel biologique choisi dans le groupe constitué par une bactérie, un extrait d'une bactérie ou un extrait d'un milieu de culture d'une bactérie.

La présente demande décrit par ailleurs l'utilisation d'un produit cosmétique pour améliorer l'apparence d'une zone de peau présentant un état de dysbiose n'entraînant aucun symptôme pathologique, ledit produit étant dépourvu d'agent conservateur, ayant un pH compris 5.5 et 6.5 et comprenant au moins 90% en masse d'un mélange constitué d'eau, de glycérol, d'au moins un polyacrylate réticulé, et d'au moins un polyol différent du glycérol.

Dans un mode de réalisation particulier, le produit comprend au moins 90% en masse d'un mélange constitué d'eau, de glycérol, d'au moins un polyacrylate réticulé, d'au moins un polyéthylèneglycol et de 1,2-octanediol. Le produit comprend de préférence ce mélange en une quantité minimale supérieure ou égale à une valeur choisie dans le groupe constitué par 90%, 95%, 96%, 97%, 98%, 99%, 99,5% et 99,9% en masse. Le produit est de préférence constitué de ce mélange.

Dans un mode de réalisation, le produit ne contient moins de 5% en masse de matériel biologique choisi dans le groupe constitué par une bactérie, un extrait d'une bactérie ou un extrait d'un milieu de culture d'une bactérie. Le produit contient de préférence une quantité de matériel biologique inférieure à une valeur choisie dans le groupe constitué par 5%, 4%, 3%, 2%, 1%, 0.5%, 0.1%, 0.05% et 0.01 % en masse. Le produit est de préférence dépourvu de tout matériel biologique.

L'ensemble de l'invention est utilisé pour le traitement d'un désordre ou d'une maladie associé à une dysbiose, c'est-à-dire associé à un déséquilibre du microbiote cutané. Le déséquilibre peut se traduire par un effet choisi dans le groupe constitué par une diminution du nombre des espèces bactériennes, une augmentation de la population d'au moins un pathogène, une diminution de la population d'au moins une bactérie commensale, une augmentation de la population d'au moins une bactérie commensale, le passage d'un caractère commensale à un caractère pathogène d'une bactérie.

La dysbiose découle d'une modification de la composition et/ou du fonctionnement du microbiote qui peut être à la fois qualitative et quantitative. La dysbiose peut se traduire par une diminution de la diversité des espèces bactériennes résidentes constituant le microbiote. La dysbiose peut également découler de l'apparition ou de l'accroissement d'une colonie d'un microorganisme pathogène qui déclenche ou aggrave une dermatose. Enfin, la dysbiose peut découler de la prolifération anormale d'une souche endogène du microbiote résident.

La dysbiose peut résulter d'une diminution ou d'une augmentation de la diversité bactérienne du microbiote cutané initial, i.e. non perturbé par la dysbiose. La dysbiose peut également résulter d'une diminution ou d'un accroissement d'une colonie bactérienne, par exemple une colonie de bactérie commensale du microbiote initial, ou une colonie d'une bactérie pathogène exogène.

La dysbiose est de préférence une pathologie qui endommage uniquement l'épiderme et ne génère pas de lésions dermiques. Le terme "épiderme" désigne la couche superficielle et non vasculaire de la peau dont l'épaisseur varie de 50 à 120 microns en moyenne, qui comprend quatre couches continues résultant de la différentiation des kératinocytes : une couche basale, une couche épineuse, une couche granulaire et une couche cornée.

La dysbiose peut être associée à une sécheresse cutanée plus ou moins importante que l'ensemble de l'invention permet de réduire de façon significative dans certains modes de réalisation. Il est connu de l'art antérieur que la couche cornée joue un rôle primordial dans le maintien du degré d'hydratation de la peau. D'une part, l'eau est fixée sur des substances hygroscopiques intracellulaires (facteurs naturels d'hydratation ou NMF) qui sont formées au cours de la kératinisation : les NMF participent à l'hydratation statique de l'épiderme. D'autre part, les lipides extracellulaires limitent la perméabilité de la peau et la perte en eau. Enfin, de l'eau provenant des capillaires sanguins migre naturellement vers la surface de la peau pour l'hydratation. Les soins hydratants de l'art antérieur adoptent deux stratégies complémentaires pour tenir compte des deux premiers mécanismes. L'hydratation cutanée apportée par ces soins est une hydratation de surface, générée grâce à des substances hydrophiles capables de retenir de l'eau (humectants) et grâce à des composés lipophiles pour limiter la perte en eau (occlusifs).

Or, il a été découvert de façon surprenante que l'ensemble de l'invention est bien plus efficace qu'un produit hydratant de l'art antérieur agissant uniquement sur le niveau d'hydratation de surface de la peau. L'ensemble de l'invention permet de renforcer le processus naturel et physiologique d'hydratation de la peau en accélérant ou en intensifiant la remontée de l'eau contenue dans les capillaires sanguins vers le derme et l'épiderme. L'ensemble de l'invention peut avantageusement constituer un produit hydratant tissulaire et non un produit hydratant de surface. De façon avantageuse, il procure un effet hydratant supérieur à celui obtenu dans l'art antérieur sans faire usage d'humectants et de composés gras occlusifs qui limitent la perte en eau, mais sans en apporter véritablement.

La dysbiose peut être une dermatose choisie dans le groupe constitué par la rosacée, le psoriasis, l'eczéma, l'hydradénite suppurée, le pityriasis, l'état pelliculaire du cuir chevelu et l'acné. Ces pathologies peuvent être accompagnées de symptômes de sécheresse cutanée plus ou moins importants.

L'ensemble de l'invention permet également de diminuer le risque de survenue des complications des dysbioses qui sont essentiellement d'origine infectieuse. Les complications observées dans l'art antérieur découlent généralement de surinfections microbiennes ou virales (déclenchement d'un herpès) ou de l'apparition d'une mycose.

La dysbiose peut être un eczéma. On entend par eczéma, au sens de l'invention, un ensemble de dermatoses érythémato-vésiculeuses prurigineuses comprenant des nappes de lésions épidermiques élémentaires qui évoluent le plus souvent en lésions dermiques. L'érythème est le plus souvent accompagné de micro-vésicules remplies d'un liquide clair. La dysbiose peut être un eczéma chronique ou un eczéma de contact. La pathologie évoluant de façon chronique ou étant déclenchée par contact peut être localisée sur une ou plusieurs zones particulières du corps telles que le visage ou les membres. L'eczéma chronique peut présenter plusieurs degrés de sévérité : pour un eczéma chronique sous forme sèche par exemple, la peau est couverte de placards rouges et croûteux, et son niveau de desquamation est variable. Sous une forme plus grave, l'eczéma dit lichénifié ou kératosique se traduit par un épaississement de la couche cornée et la formation de fissures.

L'eczéma peut être une dermatite atopique, un eczéma de forme sèche, un eczéma lichénifié, un eczéma kératosique ou un eczéma dyshidrosique. Dans le cas d'un eczéma, notamment d'une dermatite atopique, le traitement est avantageusement appliqué au moins au stade initial d'évolution de la maladie dit stade érythémateux. Le traitement permet d'éviter l'évolution de la maladie à un stade ultérieur, et notamment la formation de lésions dermiques souvent formées par le patient qui se gratte pour calmer le prurit. L'inventeur a trouvé de façon surprenante que le produit peut être utilisé pour traiter efficacement la maladie bien en amont de la phase vésiculeuse, ce qui n'avait jamais été proposé auparavant.

La dermatite atopique, forme d'eczéma la plus courante, peut évoluer sur un mode chronique ou par poussées récidivantes. Elle survient essentiellement chez des patients génétiquement prédisposés à la synthèse accrue d'immunoglobulines E (IgE). On distingue la dermatite atopique du nourrisson de celle de l'enfant. Chez le nourrisson, l'eczéma est prurigineux, érythémato-suintant et croûteux. Le front et les joues sont atteints en premier. L'éruption peut ensuite s'étendre aux oreilles et au cuir chevelu. Chez l'enfant à partir de 3 ans, des plaques lichénifiées, prurigineuses, rarement suintantes, prédominent au niveau des plis des coudes. A partir de la puberté, l'eczéma disparaît définitivement dans 90 % des cas. La dermatite atopique peut avoir un degré de sévérité plus ou moins élevé selon l'intensité et l'étendue des lésions, l'intensité des démangeaisons et l'impact sur la qualité de vie. L'homme du métier saura réaliser une évaluation plurifactorielle conformément à ses connaissances générales pour définir si la dermatite atopique du patient est légère, modérée ou sévère. Différentes échelles sont utilisées par les médecins pour évaluer cette sévérité, notamment SCORAD (Scoring atopic dermatitis), EASI (Eczema Area and Severity Index) et DLQI (Dermatology Life Quality Index).

Dans un mode de réalisation particulier, la dysbiose est une dermatite atopique au stade érythémateux.

Le déséquilibre du microbiote cutané peut découler de la présence, voire de la prédominance d'au moins Staphylococcus aureus, Propionibacterium acnes, Malassezia ou Demodex. Par exemple, la dysbiose peut être une acné dans laquelle les souches de Propionibacterium acnes retrouvées sur la zone de peau de la personne sont distinctes de celles retrouvées chez un individu sain. La dysbiose peut être également une acné découlant de la présence de Propionibacterium granulosum. La dermatite atopique peut être caractérisée par une diminution de la diversité bactérienne, et par voie de conséquence, la colonisation de la zone de peau par Staphylococcus aureus. La rosacée peut être accompagnée d'une augmentation de la population de mite Demodex.

La zone de peau atteinte d'une dysbiose peut être la peau du corps, du visage, du cou, du contour des yeux ou du cuir chevelu. La zone de peau concernée par le traitement sera de préférence la peau du visage ou du cou.

Le produit a de préférence un pH inférieur à 6.5 notamment pour préserver la sécrétion de bactériocines par les bactéries commensales de la zone de peau sur laquelle le produit est appliqué et, par voie de conséquence, limiter voire empêcher le développement de certains pathogènes.

Le pH du produit va de préférence de 5.5 à 6.5, et peut être de l'ordre de 6.0 ou égal à 6.0 (aux incertitudes de mesure près à 20 °C).

Il contient de très faibles quantités ou est dépourvu de conservateur(s) chimique(s) comme les parabens qui risquent de bloquer le renouvellement des bactéries commensales et de provoquer une allergie chez la personne. Par « très faibles quantités », on entend une quantité inférieure à la quantité usuelle, propre à chaque conservateur, et connue de l'homme du métier. Un agent conservateur au sens de la présente invention est un produit chimique de synthèse aux propriétés biocides, par exemple l'éthanol, le phénoxyéthanol, les parabens.

Le produit comprend de préférence au moins 90% en masse d'un mélange constitué d'eau, de glycérol, d'au moins un polyacrylate réticulé et d'au moins un polyol différent du glycérol. Le produit peut être fabriqué par exemple en mettant le polyacrylate réticulé en suspension dans un mélange d'eau et de polyols (incluant la glycérine).

Le mélange peut contenir au moins un polyol différent de la glycérine choisi dans le groupe constitué par les 1,2-alcane-diols comprenant de 5 à 12 atomes de carbone, de préférence de 8 à 10 atomes de carbone, par exemple le 1,2-octanediol. L'alcane-diol représente de préférence 0.01% à 10%, de préférence de 2% à 8% en masse du produit. D'autres polyols sont par exemple le propylène glycol, le 1,3-butylène glycol, le 1,8-octane diol, le 1,2-pentanediol, le mannitol ou le sorbitol.

Le ratio massique entre la glycérine et le 1,2-octanediol est de préférence compris entre 5:1 et 15:1, de préférence encore entre 8:1 et 10:1. Le polyol différent du glycérol peut être un polyéthylène glycol ou le 1,2-octanediol.

Selon une variante, le produit est uniquement constitué d'eau, du glycérol, d'au moins un polymère polyacrylate réticulé, d'au moins un polyéthylène glycol de poids moléculaire inférieur à 1000 g/mol et de 1,2-octanediol. Le polyéthylène glycol a de préférence une masse moléculaire allant de 100 à 1000 g/mol, de préférence allant de 200 à 600 g/mol. Il peut représenter de 0.05% à 1.0%, de préférence de 0.5% à 5% en masse, et de préférence encore de 1% à 5% en masse du produit. Le produit peut contenir de 0.1% à 1.5% en masse, de préférence de 0.5% à 1.5% en masse de 1,2-octanediol.

Dans un mode de mise en oeuvre, le produit est constitué d'eau, de 5% à 25 % en masse, de préférence de 5% à 15% en masse, de glycérol, de 0.01% à 2% en masse de polyacrylate réticulé; de 0.05% à 1.0%, de préférence de 0.5% à 5% en masse, et de préférence encore de 1% à 5% en masse, d'un polyéthylène glycol, et de 0.1% à 1.5% en masse, de préférence de 0.5% à 1.5% en masse de 1,2-octanediol.

Le polyacrylate réticulé peut être un composé de dénomination INCI carbomer et avoir un des numéros CAS suivants: 9007-20-9, 9003-01-4, 76050-42-5, 9062-04-8, 9007-16-3 ou 9007-17-4. Le polyacrylate réticulé est de préférence un homopolymère de l'acide acrylique réticulé avec un éther allylique du pentaérythritol ou un éther allylique du saccharose. On préfère que le taux de réticulation du polymère soit élevé.

Le polyacrylate réticulé peut être substitué, dans certaines modes de réalisation, par un acide polyacrylique, un acide polyméthacrylique, un copolymère de l'acide méthacrylique et de l'acide acrylique, un sel de l'un de ces acides obtenus par neutralisation avec une base, un ester de l'un de ces acides, ou un composé réticulé obtenu à partir de ces acides.

L'eau représente de préférence de 5% à 60% en masse, de préférence de 10% à 50% en masse et de préférence encore de 30% à 50% en masse de la masse du produit. La proportion de la glycérine peut aller de 3 % à 80 % en masse, préférentiellement de 4% à 70% en masse de la masse du produit. Le polyacrylate réticulé peut représenter de 0.01%à 2% en masse de la masse du produit, par exemple de 0.01% à 0.4% en masse de la masse du produit.

Le produit peut être sous la forme d'une lotion, d'un gel, d'une émulsion huile-dans-eau ou eau-dans-huile, de gels, pommades, sprays ou cataplasmes. Le produit est de préférence sous forme de gel ou d'hydrogel. Le produit peut comprendre un support non soluble dans l'eau, ledit support pouvant être imprégné de produit. Le support est par exemple un textile non tissé, un tricot ou une gaze. Le support peut être découpé à la forme de la zone de la peau que l'on souhaite traiter ; par exemple, le support a la forme d'un masque pour le visage. Alternativement, le produit ne comprend pas un support, et il est appliqué sur la zone de peau avant d'être recouvert d'un support.

Le produit peut comprendre en outre tout composé actif connu de l'homme du métier pouvant avoir un effet bénéfique sur le traitement de la maladie à traiter, ce composé actif n'étant pas un matériel biologique choisi dans le groupe constitué par une bactérie, un extrait d'une bactérie ou un extrait d'un milieu de culture d'une bactérie. Le produit pourra contenir au moins un actif choisi dans le groupe constitué par les agents hydratants et les molécules agissant sur l'activation des fibroblastes et des kératinocytes, notamment les actifs stimulant la sécrétion de peptides antibactériens par les kératinocytes.

Dans un mode de réalisation, le produit est avantageusement dépourvu de tout actif dermatologique et de tout conservateur chimique. Le produit est de préférence dépourvu de tout composé corticoïde et/ou de tout anti-histaminique.

La première étape d'application du produit sur la peau se fait de préférence en couche épaisse pendant une durée de 30 mn. Dans un mode de réalisation particulier, le produit est appliqué sur la peau pour former une couche épaisse, par exemple une couche d'épaisseur allant de 2 à 3 mm. La couche de produit peut être recouverte avec un tissu de gaze stérile. Le produit est de préférence laissé à agir pendant une durée égale à au moins 10 minutes, de préférence au moins 20 minutes, et de préférence encore au moins 30 minutes.

A l'issue de cette étape d'application du produit, la couche de produit et le tissu de gaze - lorsque ce dernier a été utilisé - sont de préférence retirés. Le produit peut être retiré par exemple avec un tissu de gaze stérile par essuyage.

L'étape de nettoyage peut être réalisée par essuyage ou par lavage de la zone de peau avec une lotion neutre.

Le produit est utilisé pour préparer la peau à l'application d'une composition de transplantation bactérienne comprenant au moins un matériel biologique choisi dans le groupe constitué par une bactérie, un extrait d'une bactérie ou un extrait d'un milieu de culture d'une bactérie.

Dans un mode de réalisation, le produit peut être utilisé dans le cadre d'un traitement cosmétique ou d'un traitement dermatologique comprenant une étape de transplantation d'un microbiote ou d'une fraction de microbiote, qui est réalisée de façon séparée et postérieurement à une étape de retrait du produit par nettoyage. L'étape de transplantation d'un microbiote est de préférence réalisée dès que l'étape de retrait du produit est terminée. Dans le cadre du traitement, de la personne atteinte de dysbiose la fréquence d'application du produit suivie d'une étape de transplantation de préférence sera de préférence d'au moins une fois par semaine pendant au moins un mois.

L'étape de transplantation comprend de préférence une étape de préparation d'une solution ou d'une dispersion contenant un matériel biologique, par exemple une bactérie commensale d'un microbiote soin. La solution ou la dispersion est de préférence préparée à partir d'une composition lyophilisée. Ladite composition peut prendre la forme d'une poudre ou d'un comprimé.

La transplantation du microbiote peut être réalisée par application sur la peau nettoyée d'une solution ou d'une dispersion obtenue par dissolution d'un microorganisme lyophilisé dans un milieu liquide, de préférence stérile. La solution ou la dispersion est de préférence préparée juste avant l'étape de transplantation sur la peau.

Le milieu liquide comprend ou est constitué d'eau, l'eau pouvant être une eau minérale, une eau de source ou un sérum physiologique. Le pH du milieu va de préférence de 5.5 à 6.5, par exemple est égal à 6. Le milieu fluide peut être sous la forme d'une émulsion d'une phase grasse et d'une phase aqueuse. On préfère que le milieu fluide soit exempt de tout conservateur chimique. L'eau de source est par exemple l'eau d'Avène, de La Bourboule, de La Roche-Posay, de Molitg-les-Bains, de Saint-Gervais ou d'Uriage.

Le matériel biologique peut être un extrait d'une bactérie, par exemple un lysat d'une bactérie probiotique d'un microbiote sain, ou une bactérie, par exemple une bactérie commensale d'un microbiote sain.

On peut citer comme matériel biologique les microorganismes tels que Staphylococcus epidermis, Staphylococcus haemolyticus, Staphylococcus homonis, Staphylococcus similans, Corynobacterium lipophiles, Corynobacterium jeikeium, Corynobacterium urealyticum, Corynobacterium minutissimum, Propionobacter granulosum, Propionobacter avidum, Micrococcus luteus, Micrococcus varians, Streptococcus A, Streptococcus C et Streptococcus G, Roseomonas mucosa et des espèces de Brevibacterium.

La composition de transplantation microbienne peut comprendre un probiotique à titre de matériel biologique. Un tel probiotique peut être choisi parmi des microorganismes sous forme vivante, dormante ou inactivée, un de leurs métabolites ou un de leurs fragments. Le probiotique pourra être choisi parmi les bactéries des espèces Lactobacillus et Bifidobacterium, et les levures d'espèce Saccharomyces. Il sera de préférence sous forme lyophilisée.

Le matériel biologique de la composition de transplantation microbienne peut comprendre au moins une bactérie filamenteuse non photosynthétique ou un de ses extraits. La bactérie filamenteuse peut être une bactérie du genre choisi dans le groupe constitué par Beggiatoa, Flexithix, Leucothrix, Sphaerotilus ou Vitreoscilla, par exemple Vitreoscilla filliformis (ATCC 15551), Itreoscilla beggiatoïdes (ATCC 43181), Beggiatoa alba (ATCC 33555), Flexithrix dorotheae (ATCC 23163), Leucothrix mucor (ATCC 25107) ou Sphaerotilus natans (ATCC 13338). Un extrait de ces bactéries peut désigner le surnageant du milieu de fermentation de ladite bactérie, l'enveloppe ou une fraction d'enveloppe de la dite bactérie, la biomasse obtenue par culture de ladite bactérie, un extrait obtenu par traitement de ladite biomasse et les mélanges de ces dérivés, par exemple un lysat de bactérie dans son milieu de culture.

La composition de transplantation microbienne peut également comprendre au moins un prébiotique au titre de matériel biologique et d'extrait d'un milieu de culture d'une bactérie. Les prébiotiques sont connus de l'homme du métier et permettent de stimuler la croissance ou l'activité du ou des microorganismes commensales de la composition de microorganisme(s). On préfère que le prébiotique ne soit pas métabolisable par les microorganismes responsables de la maladie à traiter. Le prébiotique pourra être choisi parmi les oligosaccharides et les polysaccharides comprenant de 2 à 100 unités osidiques. Il peut être par exemple choisi parmi le glucose, le galactose, le xylose, le maltose, le lactose, un amidon, un xylane, une hémicellulose et une inuline.

La composition peut comprendre par ailleurs tout excipient cosmétique ou pharmaceutique connu de l'homme du métier.

L'invention a pour objet un ensemble d'un produit et d'une composition pour son utilisation dans le traitement d'une dysbiose, le produit et la composition de transplantation microbienne étant tels que décrits précédemment, et étant conditionnés de façon séparée dans un même emballage. Le produit et la composition sont de préférence destinés à une application séquentielle et séparée, le produit étant destiné à être appliqué sur une zone de la peau dans une première étape, laquelle première étape est suivie d'une deuxième étape, qui consiste à appliquer la composition sur la même zone de peau.

Ainsi, l'invention concerne une association pour son utilisation dans le traitement d'une dysbiose, laquelle association est sous forme d'un produit de combinaison comprenant au moins : a) une quantité efficace d'un gel dépourvu d'agent conservateur, ayant un pH compris 5.5 et 6.5 et comprenant au moins 90% en masse d'un mélange constitué d'eau, de glycérol, d'au moins un polyacrylate réticulé, et d'au moins un polyol différent du glycérol, et b) une quantité efficace d'un matériel biologique, ledit gel et ledit matériel biologique étant utilisés de façon séquentielle et séparée dans le temps.

Les caractéristiques du gel et du matériel biologique faisant partie de l'ensemble ou de l'association mentionnée précédemment sont identiques aux caractéristiques qui ont été décrites plus haut dans la description en relation avec le produit dépourvu de conservateur et l'ensemble de ce produit et d'une composition de transplantation bactérienne, si bien que leur description n'est pas reproduite une deuxième fois dans cette partie.

Dans le cadre de l'invention, le gel et le matériel biologique ne font pas partie de la même composition et ne forment pas un même produit, si bien qu'ils ne sont pas appliqués simultanément sur une zone de peau atteinte d'une dysbiose pathologique ou d'une dysbiose non pathologique. L'inventeur a démontré de manière surprenante que l'administration du gel sur une zone de peau, suivie de l'administration après un certain délai allant par exemple de quelques minutes à quelques heures, d'un matériel biologique sur la même zone de peau permet de traiter une dysbiose de nature pathologique et une dysbiose de nature non pathologique. On préfère que le gel soit éliminé de la surface de la zone de peau, par exemple par essuyage, avant s'administrer le matériel biologique. Le délai entre l'application du gel et l'application subséquente du matériel biologique sur la zone de peau atteinte, va de préférence de 5 minutes à 1 heures, de préférence de 10 minutes à 50 minutes et de préférence encore de 20 minutes à 40 minutes. L'essuyage est réalisé dans des conditions stériles, de manière à éviter toute contamination de la peau par des microbes, de manière à ce que la peau - nettoyée et dépourvue de toute flore microbienne en surface -puisse recevoir un matériel biologique exogène apte à régénérer une flore bactérienne à la surface de la peau permettant son fonctionnement biologique normal.

La composition de transplantation microbienne est de préférence sous une forme lyophilisée dans l'ensemble. L'ensemble peut dans ce cas également comprendre un troisième produit qui est sous forme fluide et qui peut servir à la dissolution ou à la dispersion de la composition de transplantation microbienne), lorsque celle-ci se trouve sous forme lyophilisée.

L'ensemble de l'invention peut être utilisé dans le cadre d'une méthode de soin cosmétique d'un sujet souhaitant améliorer l'esthétique et/ou le confort de sa peau non atteinte par une pathologie. Le produit permet de rééquilibrer, de contrôler, de maintenir ou de moduler le microbiote cutané de la peau saine, lorsque celui-ci est perturbé de façon occasionnelle par un déclencheur chimique ou physique, tel qu'une exposition inhabituelle aux UV, à la chaleur ou à un agent chimique toxique. La méthode de soin cosmétique ne s'applique pas à un sujet chez lequel l'équilibre et/ou le fonctionnement du microbiote cutané est perturbé de façon chronique et chez lequel la modification du microbiote déclenche une maladie.

La présente demande concerne enfin l'utilisation d'un produit tel que décrit précédemment pour améliorer l'efficacité d'une composition comprenant du matériel biologique, dans le traitement par voie topique d'une zone de peau atteinte d'une dysbiose.

L'invention est illustrée par l'exemple suivant.

### EXEMPLE : Etude clinique pour le traitement de la dermatite atopique

Une étude est menée sur des personnes adultes âgées de 18 à 55 ans souffrant de dermatite atopique modérée (SCORAD 25-50) au stade érythémateux. Trois groupes de 10 personnes sont constitués de façon aléatoire, et chaque groupe subit un des trois traitements décrits.

### Produits appliqués

Un gel disponible dans le commerce sous la marque Osmosoft^{®}, est appliqué sur le visage et le cou des 10 personnes du premier groupe 2 fois par jour pendant 15 jours.

Un produit disponible dans le commerce comprenant 5% d'un autolysat bactérien Vitreoscilla Filiformis est appliqué dans les mêmes conditions, sur le visage et le cou de 10 autres personnes du deuxième groupe présentant les mêmes symptômes, 2 fois par jour pendant 15 jours.

Sur les 10 personnes du troisième groupe, on suit le protocole consistant à appliquer successivement le gel et le produit de l'art antérieur, tous deux conformes à ceux qui ont été utilisés dans les deux essais précédents. Deux fois par jour pendant 15 jours, 10 personnes sont soignées de la façon suivante : le gel est appliqué en cataplasme pendant 30 minutes. Il est ensuite éliminé par nettoyage avec une compresse stérile. Le produit de l'art antérieur est ensuite appliqué sur les mêmes zones.

### Critères d'évaluations

Trois critères révélateurs de rémission sont évalués.

La diminution de la sécheresse cutanée (sensation de tiraillement, rougeurs, sensation rugueuse au toucher, desquamation, craquelure) des zones atteintes par la dermatite est évaluée cliniquement par un dermatologue avant le début du traitement et le dernier jour du traitement.

La diminution des démangeaisons est évaluée sur la base de déclarations des patients conformément à l'échelle d'évaluation numérique du prurit (NRS). Chaque patient devait répondre à la question suivante: "Sur une échelle de 0 à 10, 0 étant" pas de démangeaisons "et 10," pire démangeaisons imaginables ", comment évalueriez-vous vos démangeaisons au pire moment des 24 dernières heures?". La moyenne des notes obtenues le jour précédent le début du traitement et la moyenne des notes obtenues à la fin du traitement sont ensuite calculée pour chacun des trois groupes. La différence entre la note moyenne avant traitement et la note moyenne après traitement ramenée à la note moyenne avant traitement permet de calculer le pourcentage moyen de diminution des démangeaisons pour chaque groupe.

L'hydratation de la peau est mesurée à l'aide d'un cornéomètre CM 825 dans les 7 heures suivant l'application du gel. L'augmentation du taux d'hydratation est calculée à l'issue du traitement sous la forme d'un pourcentage.

### Résultats

### 3-1 Tests Dermatologiques

Les résultats sont présentés dans le Tableau 1.

### [Table 1]

**Tableau 1**

| Pourcentage d'évolution par rapport à l'état initial | Produit de l'art antérieur N°1 | Produit de l'art antérieur N°2 | Invention |
|---|---|---|---|
| Sécheresse cutanée | -70% | -65% | -80% |
| Démangeaisons | -75% | -55% | -85% |

Il apparait une diminution significative (p<0.03) de la sécheresse cutanée et des démangeaisons de la peau chez les sujets traités avec le produit de l'invention.

### 3-2 Tests d'hydratation

Les résultats sont présentés dans le Tableau 2.

### [Table 2]

**Tableau 2**

| | Invention | Produit de l'art antérieur n°1 | Produit de l'art antérieur N°2 |
|---|---|---|---|
| Temps (heures) | Pourcentage d'augmentation (%) | | |
| 1 | 80 | 70 | 55 |
| 2 | 78 | 65 | 50 |
| 3 | 76 | 60 | 45 |
| 4 | 74 | 57 | 42 |
| 5 | 72 | 57 | 40 |
| 6 | 70 | 56 | 38 |
| 7 | 74 | 55 | 36 |

L'état d'hydratation de la peau est amélioré par le traitement de l'invention.

## Revendications

1. Ensemble d'un produit et d'une composition conditionnés de façon séparée, pour son utilisation dans le traitement par voie topique d'une zone de peau d'une personne atteinte d'une dysbiose:
- ledit produit étant dépourvu d'agent conservateur, ayant un pH compris 5.5 et 6.5 et comprenant au moins 90% en masse d'un mélange constitué d'eau, de glycérol, d'au moins un polyacrylate réticulé, et d'au moins un polyol différent du glycérol,
- ladite composition étant une composition de transplantation bactérienne comprenant au moins un matériel biologique choisi dans le groupe constitué par une bactérie, un extrait d'une bactérie ou un extrait d'un milieu de culture d'une bactérie.

2. Ensemble d'un produit et d'une composition conditionnés de façon séparée, pour son utilisation selon la revendication 1, **caractérisé en ce que** l'extrait d'une bactérie est un lysat d'une bactérie probiotique d'un microbiote cutané sain.

3. Ensemble d'un produit et d'une composition conditionnés de façon séparée, pour son utilisation selon la revendication 1, **caractérisé en ce que** la bactérie est une bactérie commensale d'un microbiote cutané sain.

4. Ensemble d'un produit et d'une composition conditionnés de façon séparée, pour son utilisation selon la revendication 1, **caractérisé en ce que** le polyol est un polyéthylèneglycol ou le 1,2-octanediol.

5. Ensemble d'un produit et d'une composition conditionnés de façon séparée, pour son utilisation selon la revendication 1, **caractérisé en ce que** la dysbiose résulte d'une diminution ou d'une augmentation de la diversité bactérienne du microbiote cutané initial.

6. Ensemble d'un produit et d'une composition conditionnés de façon séparée, pour son utilisation selon la revendication 1, **caractérisé en ce que** la dysbiose résulte d'une diminution ou d'un accroissement d'une colonie bactérienne.

7. Ensemble d'un produit et d'une composition conditionnés de façon séparée, pour son utilisation selon la revendication 6, **caractérisé en ce que** la colonie bactérienne est une colonie de bactérie commensale du microbiote initial.

8. Ensemble d'un produit et d'une composition conditionnés de façon séparée, pour son utilisation selon la revendication 6, **caractérisé en ce que** la colonie bactérienne est une colonie d'une bactérie pathogène exogène.

9. Ensemble d'un produit et d'une composition conditionnés de façon séparée, pour son utilisation selon la revendication 1, **caractérisé en ce que** la dysbiose est une dermatose choisie dans le groupe constitué par la rosacée, le psoriasis, l'eczéma, l'hydradénite suppurée, le pityriasis, l'état pelliculaire du cuir chevelu et l'acné.

10. Ensemble d'un produit et d'une composition conditionnés de façon séparée, pour son utilisation selon la revendication 9, **caractérisé en ce que** la pathologie est une dermatite atopique au stade érythémateux.

11. Ensemble d'un produit et d'une composition conditionnés de façon séparée, pour son utilisation selon la revendication 1, **caractérisé en ce que** le matériel biologique est choisi dans le groupe constitué par Staphylococcus epidermis, Staphylococcus haemolyticus, Staphylococcus homonis, Staphylococcus similans, Corynobacterium lipophiles, Corynobacterium jeikeium, Corynobacterium urealyticum, Corynobacterium minutissimum, Propionobacter granulosum, Propionobacter avidum, Micrococcus luteus, Micrococcus varians, Streptococcus A, Streptococcus C et Streptococcus G, Roseomonas mucosa et Brevibacterium.

12. Ensemble d'un produit et d'une composition conditionnés de façon séparée, pour son utilisation selon la revendication 1, **caractérisé en ce que** le produit comprend un support non soluble dans l'eau.

13. Ensemble d'un produit et d'une composition conditionnés de façon séparée, pour son utilisation selon la revendication 13, **caractérisé en ce que** le support a la forme d'un masque pour le visage.

14. Ensemble d'un produit et d'une composition conditionnés de façon séparée, pour son utilisation selon la revendication 1, **caractérisé en ce qu'**il comprend de 5% à 60% en masse d'eau, de 5% à 25% en masse de glycérol, de 0.05% à 1.0% en masse d'au moins un polyéthylène glycol, de 0.1% à 1.5% en masse de 1,2-octanediol et de 0.01% à 2% en masse d'au moins un polymère polyacrylate réticulé, les pourcentages en masse étant exprimés par rapport à la masse totale du produit, et la somme des pourcentages allant de 90% à 100%.

## Patentansprüche

1. Einheit eines Produkts und einer Zusammensetzung, die auf getrennte Weise konditioniert sind, für ihre Verwendung bei der Behandlung auf topischem Weg eines Bereichs der Haut einer Person, die von einer Dysbiose befallen ist:
- wobei das Produkt kein Konservierungsmittel enthält, einen PH im Bereich zwischen 5,5 und 6,5 aufweist und mindestens 90 Massenprozent einer Mischung umfasst, die aus Wasser, Glycerol, mindestens einem vernetzten Polyacrylat und mindestens einem vom Glycerol verschiedenen Polyol besteht,
- wobei die Zusammensetzung eine Zusammensetzung zur Bakterientransplantation ist, umfassend mindestens ein biologisches Material, ausgewählt aus der Gruppe, bestehend aus einem Bakterium, einem Extrakt eines Bakteriums oder einem Extrakt eines Nährbodens eines Bakteriums.

2. Einheit eines Produkts und einer Zusammensetzung, die auf getrennte Weise konditioniert sind, für ihre Verwendung nach Anspruch 1, **dadurch gekennzeichnet, dass** der Extrakt eines Bakteriums ein Lysat eines probiotischen Bakteriums einer gesunden Hautmikroflora ist.

3. Einheit eines Produkts und einer Zusammensetzung, die auf getrennte Weise konditioniert sind, für ihre Verwendung nach Anspruch 1, **dadurch gekennzeichnet, dass** das Bakterium ein kommensales Bakterium einer gesunden Hautmikroflora ist.

4. Einheit eines Produkts und einer Zusammensetzung, die auf getrennte Weise konditioniert sind, für ihre Verwendung nach Anspruch 1, **dadurch gekennzeichnet, dass** das Polyol ein Polyethylenglykol oder 1,2-Octandiol ist.

5. Einheit eines Produkts und einer Zusammensetzung, die auf getrennte Weise konditioniert sind, für ihre Verwendung nach Anspruch 1, **dadurch gekennzeichnet, dass** sich die Dysbiose aus einer Verkleinerung oder einer Vergrößerung der bakteriellen Diversität der anfänglichen Hautmikroflora ergibt.

6. Einheit eines Produkts und einer Zusammensetzung, die auf getrennte Weise konditioniert sind, für ihre Verwendung nach Anspruch 1, **dadurch gekennzeichnet, dass** sich die Dysbiose aus einer Verkleinerung oder einer Vergrößerung einer Bakterienkultur ergibt.

7. Einheit eines Produkts und einer Zusammensetzung, die auf getrennte Weise konditioniert sind, für ihre Verwendung nach Anspruch 6, **dadurch gekennzeichnet, dass** die Bakterienkultur eine Kultur des kommensalen Bakteriums der anfänglichen Hautmikroflora ist.

8. Einheit eines Produkts und einer Zusammensetzung, die auf getrennte Weise konditioniert sind, für ihre Verwendung nach Anspruch 6, **dadurch gekennzeichnet, dass** die Bakterienkultur eine Kultur eines exogenen pathogenen Bakteriums ist.

9. Einheit eines Produkts und einer Zusammensetzung, die auf getrennte Weise konditioniert sind, für ihre Verwendung nach Anspruch 1, **dadurch gekennzeichnet, dass** die Dysbiose eine Dermatose ist, ausgewählt aus der Gruppe, bestehend aus Rosacea, Psoriasis, Ekzem, Hidradenitis suppurativa, Pityriasis versicolor, Schuppen der Kopfhaut und Akne.

10. Einheit eines Produkts und einer Zusammensetzung, die auf getrennte Weise konditioniert sind, für ihre Verwendung nach Anspruch 9, **dadurch gekennzeichnet, dass** die Pathologie eine atopische Dermatitis in erythematösem Stadium ist.

11. Einheit eines Produkts und einer Zusammensetzung, die auf getrennte Weise konditioniert sind, für ihre Verwendung nach Anspruch 1, **dadurch gekennzeichnet, dass** das biologische Material ausgewählt ist aus der Gruppe, bestehend aus Staphylococcus epidermis, Staphylococcus haemolyticus, Staphylococcus homonis, Staphylococcus similans, Corynobacterium lipophiles, Corynobacterium jeikeium, Corynobacterium urealyticum, Corynobacterium minutissimum, Propionobacter granulosum, Propionobacter avidum, Micrococcus luteus, Micrococcus varians, Streptococcus A, Streptococcus C und Streptococcus G, Roseomonas mucosa und Brevibacterium.

12. Einheit eines Produkts und einer Zusammensetzung, die auf getrennte Weise konditioniert sind, für ihre Verwendung nach Anspruch 1, **dadurch gekennzeichnet, dass** das Produkt einen wasserunlöslichen Träger umfasst.

13. Einheit eines Produkts und einer Zusammensetzung, die auf getrennte Weise konditioniert sind, für ihre Verwendung nach Anspruch 13, **dadurch gekennzeichnet, dass** der Träger die Form einer Gesichtsmaske aufweist.

14. Einheit eines Produkts und einer Zusammensetzung, die auf getrennte Weise konditioniert sind, für ihre Verwendung nach Anspruch 1, **dadurch gekennzeichnet, dass** sie von 5 bis 60 Massenprozent Wasser, von 5 bis 25 Massenprozent Glycerol, von 0,05 bis 1,0 Massenprozent mindestens eines Polyethylenglykols, von 0,1 bis 1,5 Massenprozent 1,2-Octandiol und von 0,01 bis 2 Massenprozent mindestens eines vernetzten Polyacrylatpolymers umfasst, wobei die Massenprozentsätze mit Bezug auf die Gesamtmasse des Produkts exprimiert sind und die Summe der Prozentsätze von 90 % bis 100 % reicht.

## Claims

1. A separately packaged set of a product and a composition for use in the topical treatment of an area of skin of a person suffering from dysbiosis:
- said product being free of a preservative, having a pH between 5.5 and 6.5 and comprising at least 90% by mass of a mixture consisting of water, glycerol, at least one crosslinked polyacrylate, and at least one polyol different from glycerol,
- said composition being a bacterial transplantation composition comprising at least one biological material selected from the group consisting of a bacterium, an extract of a bacterium or an extract of a culture medium of a bacterium.

2. The separately packaged set of a product and a composition for use according to claim 1, **characterized in that** the extract of a bacterium is a lysate of a probiotic bacterium of a healthy skin microbiota.

3. The separately packaged set of a product and a composition for use according to claim 1, **characterized in that** the bacterium is a commensal bacterium of a healthy skin microbiota.

4. The separately packaged set of a product and a composition for use according to claim 1, **characterized in that** the polyol is polyethylene glycol or 1,2-octanediol.

5. The separately packaged set of a product and a composition for use according to claim 1, **characterized in that** the dysbiosis results from a decrease or increase in the bacterial diversity of the initial skin microbiota.

6. The separately packaged set of a product and a composition for use according to claim 1, **characterized in that** the dysbiosis results from a decrease or increase in a bacterial colony.

7. The separately packaged set of a product and a composition for use according to claim 6, **characterized in that** the bacterial colony is a commensal bacterial colony of the initial microbiota.

8. The separately packaged set of a product and a composition for use according to claim 6, **characterized in that** the bacterial colony is a colony of an exogenous pathogenic bacterium.

9. The separately packaged set of a product and a composition for use according to claim 1, wherein the dysbiosis is a dermatosis selected from the group consisting of rosacea, psoriasis, eczema, hidradenitis suppurativa, pityriasis, dandruff and acne.

10. The separately packaged set of a product and a composition for use according to claim 9, **characterized in that** the pathology is atopic dermatitis in the erythematous stage.

11. The separately packaged set of a product and a composition for use according to claim 1, **characterized in that** the biological material is selected from the group consisting of *Staphylococcus epidermis, Staphylococcus haemolyticus, Staphylococcus homonis, Staphylococcus similans, Corynebacterium lipophilic, Corynebacterium jeikeium, Corynebacterium urealyticum, Corynebacterium minutissimum, Propionobacter granulosum, Propionobacter a vidum, Micrococcus luteus, Micrococcus varians, Streptococcus* A, *Streptococcus* C and *Streptococcus* G, *Roseomonas mucosa* and *Brevibacterium.*

12. The separately packaged set of a product and a composition for use according to claim 1, **characterized in that** the product comprises a water-insoluble support.

13. The separately packaged set of a product and a composition for use according to claim 13, **characterized in that** the support is in the form of a face mask.

14. The separately packaged set of a product and a composition for use according to claim 1, **characterized in that** it comprises from 5% to 60% by mass of water, from 5% to 25% by mass of glycerol, from 0.05% to 1.0% by mass of at least one polyethylene glycol, from 0.1% to 1.5% by mass of 1,2-octanediol and 0.01% to 2% by mass of at least one crosslinked polyacrylate polymer, the percentages by mass being expressed in relation to the total mass of the product, and the sum of the percentages ranging from 90% to 100%.
